# EUROPEAN PATENT SPECIFICATION

(11) **EP 2 714 199 B1**
(45) Date of publication and mention of the grant of the patent: **19.04.2017**
(21) Application number: 12718555.1
(22) Date of filing: 24.05.2012
(51) Int. Cl.: A61Q 5/12, A61K 8/31, A61K 8/891, A61K 8/898

(54) **CLEAR HAIR CARE COMPOSITION COMPRISING BASE OIL AND SILICONE**
TRANSPARENTES HAARPFLEGEMITTEL ENTHALTEND EINE ÖLIGE GRUNDLAGE UND EIN SILICON
COMPOSITION TRANSPARENTE DE SOINS CAPILLAIRES COMPRENANT UNE HUILE DE BASE ET UN SILICONE

(30) Priority: 03.06.2011 US 201161492844 P
(43) Date of publication of application: 09.04.2014
(73) Proprietor: The Procter & Gamble Company, Cincinnati, OH 45202 (US)
(72) Inventor: OKU, Taisuke, Kobe Hyogo 657-0016 (JP); UEHARA, Nobuaki, Singapore 649823 (SG); HASEGAWA, Jun, Kobe Hyogo 658-0063 (JP)
(74) Representative: Sauvaître, Thibault Bruno
(86) International application number: PCT/US2012/039357
(87) International publication number: WO 2012/166519

(56) References cited:
- JP-A- H02 172 905
- JP-A- 2007 332 132
- JP-A- 2009 221 143
- JP-A- 2009 242 258
- JP-A- 2011 207 865
- US-A1- 2005 063 934
- US-A1- 2011 123 472
- YEON HEE LIM ET AL: "Hair conditioning effect of amino silicone softeners in varied treatment conditions", FIBERS AND POLYMERS,, vol. 11, no. 3, 1 June 2010 (2010-06-01), pages 507-515, XP009177979,

## Description

### FIELD OF THE INVENTION

The present invention relates to a non-aqueous hair care composition comprising by weight: (a) from 65% to 99.9% of a base oil consisting of: an volatile isoparaffin; a first non-volatile mineral oil having a lower viscosity and density as defined in claim 1 ; and a second non-volatile mineral oil having a higher viscosity and density as defined in claim 1 ; and (b) from 0.1% to 15% of a non-volatile silicone conditioning agent. as defined in claim 1, and 1 % or less water by weight of the composition.

### BACKGROUND OF THE INVENTION

Human hair becomes soiled due to its contact with the surrounding environment and from the sebum secreted by the scalp. The soiling of hair causes it to have a dirty feel and an unattractive appearance. The soiling of the hair necessitates shampooing with frequent regularity.

Shampooing cleans the hair by removing excess soil and sebum. However, shampooing can leave the hair in a wet, tangled, and generally unmanageable state. Once the hair dries, it is often left in a dry, rough, lusterless, or frizzy condition due to removal of the hair's natural oils and other natural conditioning and moisturizing components. The hair can further be left with increased levels of static upon drying, which can interfere with combing and result in a condition commonly referred to as "fly-away hair," or contribute to an undesirable phenomenon of "split ends." Further, chemical treatments, such as perming, bleaching, or coloring hair, can also damage hair and leave it dry, rough, lusterless, and damaged.

A variety of approaches have been developed to condition the hair. A common method of providing conditioning benefits to the hair is through the use of application of hair conditioning composition. Conditioning formulations can be in the form of rinse-off products or leave-on products, and can be in the form of an emulsion, cream, gel, spray, mousse, oil, liquid and serum.

One of the leave-on products is a hair oil products which comprises a larger amount of oily compounds and is often non-aqueous liquid product. For example, WO 2010/087004 relates to oily hair cosmetics comprising an ester compound of dibasic acid, and one or more oils selected from the group consisting of a volatile oil, an ester oil, a hydrocarbon oil, an animal or plant oil and a silicone oil. The oil hair cosmetics are said to have a high improving effect on damaged hair and excellent feeling upon use. This WO publication discloses in Example 1 a hair oil comprising 5.0% succinic acid bis-diethyleneglycol ethyl ether, 45% isododecane, 3.0% isopropyl palmitate, 10% mineral oil, 10% cyclomethicone, 2.5% dimethicone (10mPa·s), and 20% ethanol. The WO publication also discloses in Example 3 a hair oil comprising 15% succinic acid bis-diethyleneglycol ethyl ether, 40% isohexadecane, 20.45% mineral oil, 2% squalane, and 3% camellia oil.

JP2009221143 discloses a non-aqueous hair oil comprising a volatile isoparaffin, light liquid paraffin and a non-volatile silicone.

There was a need for such compositions to provide improved dry conditioning benefit. One of the methods to improve dry conditioning benefit can be the addition of non-volatile silicone conditioning agent.

However, it has been surprisingly found by the inventors of the present invention that, when non-volatile silicone conditioning agent are added, foams are observed in some hair oil compositions, especially during transportation and/or usage. Such foaming is not desirable for some consumers who consider such foaming as a signal of cleansing benefit. Thus, there is a need for such hair oil compositions to provide dry conditioning benefit while reducing such foaming.

There is also a need for such hair oil compositions to provide conditioned feel and reduced stickiness/greasiness, and to have a clear product appearance.

None of the existing art provides all of the advantages and benefits of the present invention.

### SUMMARY OF THE INVENTION

The present invention is directed to a non-aqueous hair care composition comprising by weight:
from 65% to 99.9% of a base oil consisting of:
   a volatile isoparaffin;
   a first non-volatile mineral oil having a viscosity of from 5mm²·s⁻¹ to 30mm²·s⁻¹ at 37.8°C and a relative density of from 0.80 to 0.84 at 25°C; and
   a second non-volatile mineral oil having a viscosity of from 45mm²·s⁻¹ to 100mm²· s⁻¹ at 37.8°C and a relative density of from 0.847 to 0.89 at 25°C: and
   from 0.1% to 15% of a non-volatile silicone conditioning agent having a viscosity of from 200 mm²·s⁻¹ at 25°C.
   and 1% or less of water by weight of the composition.

The hair care compositions of the present invention provide reduced foaming while providing improved dry conditioning by containing silicone conditioning agent. The hair care compositions of the present invention also provides conditioned feel and reduced stickiness/greasiness, and have a clear product appearance.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### COMPOSITION

The composition of the present invention is a non-aqueous hair care composition comprising by weight:
from 65% to 99.9% of a base oil consisting of:
   a volatile isoparaffin;
   a first non-volatile mineral oil having a viscosity of from 5mm²·s⁻¹ to 30mm²·s⁻¹ at 37.8°C and a relative density of from 0.80 to 0.84 at 25°C; and
   a second non-volatile mineral oil having a viscosity of from 45mm²·s⁻¹ to 100mm². s⁻¹ at 37.8°C and a relative density of from 0.847 to 0.89 at 25°C; and
   from 0.1% to 15% of a non-volatile silicone conditioning agent having a viscosity of from 200 mm²·s⁻¹ at 25°C.

It is believed that, by this specific base oil, the composition of the present invention has a reduced foaming while containing silicone conditioning agent, and provides a balance between reduced sticky/greasy feel and conditioned feel which could be delivered by a higher viscosity, and has a clear product appearance, compared to other base oils such as those without one of the above volatile isoparaffin, first non-volatile mineral oil, and second non-volatile mineral oil.

It is also believed that the use of the first/second non-volatile mineral oils having lower/higher viscosities and densities provides reduced foaming while solubilizing the silicone conditioning agent, and also provide improved stability especially improved silicone solubility at lower temperature, compared to the use of non-volatile mineral oil having a in-between viscosity and density.

### Non aqueous composition

The composition of the present invention is a non-aqueous composition. Non-aqueous composition herein means that the composition is substantially free of water. In the present invention, "the composition being substantially free of water" means that: the composition is free of water; or, if the composition contains water, the level of water is very low. In the present invention, the level of water, if included, 1% or less, preferably 0.5% or less, more preferably 0.3% or less, still more preferably 0.1% or less, even more preferably 0% by weight of the composition.

### Turbidity

The composition of the present invention has a homogeneous clear product appearance, i.e., homogeneous transparent product appearance.

Preferably, the composition of the present invention has a turbidity of 2.0NTU or less, 1.5NTU or less, more preferably 1.0NTU or less, still more preferably 0.7NTU or less at 25°C. Further preferably, the composition of the present invention has such above turbidity at 25°C, even after its storage at 5 °C for at least 1 hour. The transmittances are measured by using HACH 2100N Turbidimeter.

In view of homogeneous clear product appearance, it is preferred to control the level of components which are substantially insoluble to the composition of the present invention. By "substantially insoluble" compound, what is meant is that:
the compound is substantially insoluble in the compositions at the level used; and,
when containing the compounds at the level used, the compositions are either: (i) nonhomogeneous by, for example, phase separation; or (ii) homogeneous but with a higher turbidity, i.e., turbidity of above 2.0NTU (excluding 2.0NTU), preferably above 1.5NTU (excluding 1.5NTU), more preferably above 1.0NTU (excluding 1.0NTU), still more preferably above 0.7NTU (excluding 0.7NTU) at 25°C.

Such substantially insoluble compounds further include fatty compounds including, for example: fatty alcohols having a melting point of 25°C or more, such as cetyl alcohol and stearyl alcohol; fatty acids having a melting point of 25°C or more, such as stearic acid; fatty alcohol derivatives and fatty acid derivatives such as esters and ethers thereof, which derivatives have a melting point of 25°C or more; and mixtures thereof.

More preferably, the compositions of the present invention are substantially free of such substantially insoluble compounds. In the present invention, the compositions being "substantially free" of substantially insoluble compounds means that: the composition is free of substantially insoluble compounds; or, if the composition contains substantially insoluble compounds, the level of such substantially insoluble compounds is very low. In the present invention, the level of such substantially insoluble compounds is, if included, 1.0% or less, preferably 0.5% or less, more preferably 0.3% or less, still more preferably 0.1% or less, even more preferably 0%.

### Substantially free of surfactants

Preferably, the composition of the present invention is substantially free of surfactants, in view of product clarity and/or reduced undesirable foaming of the product.

Such surfactants include: anionic surfactants; cationic surfactants; amphoteric surfactants; zwitterionic surfactants; nonionic surfactants having a HLB of 5 or more; and mixtures thereof. Such nonionic surfactants having a HLB of 5 or more includes, for example: ethers of fatty alcohols such as ethers of lauryl alcohol including Laureth-3 (having a HLB of about 8); esters of fatty alcohols such as esters of lauryl alcohols.

In the present invention, "the composition being substantially free of surfactants" means that: the composition is free of surfactants; or, if the composition contains surfactants, the level of such surfactants is very low. In the present invention, the total level of such surfactants is, if included, preferably 0.1% or less, more preferably 0.01% or less, still more preferably 0.001% or less, even more preferably 0.0005% by weight of the composition.

### BASE OIL

The composition of the present invention comprises a base oil. The base oil is included at a level by weight of the composition of, from about 65% to about 99.9%, from about 70% to about 99.8%, more preferably from about 75% to about 99.5%, still more preferably from about 80% to about 99%, still more preferably from about 85% to about 98%.

The base oil useful herein consists of: a volatile isoparaffin; a first non-volatile mineral oil; and a second non-volatile mineral oil, which are explained below in detail.

In the present invention, it is preferred that the weight ratio of the volatile isoparaffin to a sum of the first and second non-volatile mineral oils is in the range of from about 1:0.2 to about 1:2, more preferably from about 1:0.5 to about 1:1.5, still more preferably from about 1: 0.75 to about 1:1.2.

In the present invention, it is preferred that the weight ratio of the first non-volatile mineral oil to the second non-volatile mineral oil is in the range of from about 1:0.05 to about 1:5, more preferably from about 1:0.1 to about 1:2, still more preferably from about 1:0.2 to about 1:1.5, even more preferably from about 1:0.2 to about 1:0.8.

### Volatile isoparaffin

The volatile isoparaffin is included at a level by weight of the composition of, preferably from about 30% to about 90%, more preferably from about 40% to about 75%, still more preferably from about 40% to about 60%.

The volatile isoparaffin useful herein have a viscosity of, preferably from about 0.5mm²· s⁻¹ to about 50mm²· s⁻¹, more preferably from about 0.8mm²· s⁻¹ to about 40mm²· s⁻¹, still more preferably from about 1mm²· s⁻¹ to about 30mm²· s⁻¹, even more preferably from about 1.5mm². s⁻¹ to about 20mm²· s⁻¹, further more preferably from about 1.5mm²· S⁻¹ to about 10mm²· s⁻¹, at 37.8°C. When using two or more isoparaffin hydrocarbon solvents, it is preferred that the mixture of isoparaffin hydrocarbon solvents have the above viscosity.

Preferably, the volatile isoparaffin useful herein are those having from about 8 to about 20 carbon atoms, more preferably from about 8 to about 16 carbon atoms, still more preferably from about 8 to about 12 carbon atoms, even more preferably from about 10 to about 12 carbon atoms.

In the present invention, preferred volatile isoparaffin include, for example, trimer, tetramer, and pentamer of isobutene, and mixtures thereof. Commercially available isoparaffin hydrocarbons may have distributions of its polymerization degree, and may be mixtures of, for example, trimer, tetramer, and pentamer. What is meant by tetramer herein is that a commercially available isoparaffin hydrocarbons in which tetramer has the highest content, i.e., tetramer is included at a level of preferably 70% or more, more preferably 80% or more, still more preferably 85% or more.

### First non-volatile mineral oils

The first non-volatile mineral oil is included at a level by weight of the composition of, preferably from about 5% to about 55%, more preferably from about 15% to about 50%, still more preferably from about 25% to about 45%, even more preferably from about 30% to about 40%.

The first non-volatile mineral oils useful herein have a viscosity of from about 5mm²· s⁻¹ to about 30mm²· s⁻¹, preferably from about 8mm²· s⁻¹ to about 20mm². s⁻¹, more preferably from about 10mm²· s⁻¹ to about 15mm²· s⁻¹, at 37.8°C. The first non-volatile mineral oils useful herein also have a relative density of from about 0.80 to about 0.84, preferably from about 0.81 to about 0.836, more preferably from about 0.815 to about 0.834, at 25°C.

### Second non-volatile mineral oils

The second non-volatile mineral oil is included at a level by weight of the composition of, preferably from about 1% to about 25%, more preferably from about 5% to about 20%, still more preferably from about 7% to about 18%.

The second non-volatile mineral oils useful herein have a viscosity of from about 45 mm² ·s⁻¹ to about 100mm²· s⁻¹, preferably from about 55mm²· s⁻¹ to about 85mm². s⁻¹, more preferably from about 65mm²· s⁻¹ to about 75mm²· s⁻¹, at 37.8°C. The second non-volatile mineral oils useful herein also have a relative density of from about 0.847 to about 0.89, preferably from about 0.85 to about 0.885, more preferably from about 0.850 to about 0.875, at 25°C.

### ESTERS

The compositions of the present invention may further contain an ester. The ester can be included in the composition by weight of, from about 0.1% to about 25%, preferably from about 0.5% to about 15%, more preferably from about 1% to about 10%, still more preferably from about 1% to about 5%, in view of the balance between conditioned feel and product stability.

The esters useful herein are those having an HLB of 3 or less, and liquid at room temperature. The esters useful herein are, for example, those selected from the group consisting of methyl esters of C12-18 fatty acids and mixtures thereof. Such esters include, for example, methyl palmitate, methyl stearate, methyl oleate, methyl linoleate, and methyl laurate. Among them, methyl stearate is preferably used in the present invention. Such esters are believed to help the silicone conditioning agents dissolved into the base oil of the present invention.

### SILICONE CONDITIONING AGENT

The composition of the present invention comprises a silicone conditioning agent. The silicone conditioning agent is included at a level by weight of the composition of, from about 0.1%, preferably from about 0.2%, more preferably from about 0.5% in view of providing dry conditioning benefit such as reduced friction, and to about 15%, preferably to about 10%, more preferably to about 8%, still more preferably to about 5% in view of conditioning benefits and/or product clarity.

The silicone conditioning agents useful herein are non-volatile, and preferably liquid at 25°C. The silicone conditioning agents useful herein have an apparent viscosity at 25°C of, from about 200mm²· s⁻¹, preferably from about 200 to about 300,000mm²· s⁻¹, more preferably from about 200 to about 50,000mm²· s⁻¹, in view of providing conditioning benefits.

The silicone conditioning agents useful herein are hydrophobic and those having a HLB of about 8 or less, preferably 5 or less.

The silicone conditioning agents useful herein are, preferably, in non-emulsion form.

### Aminosilicone

In the present invention, the silicone conditioning agent is preferably an aminosilicone. Preferably, aminosilicones useful herein have an amine content of less than about 0.12 m mol/g, more preferably less than about 0.1 m mol/g, still more preferably less than about 0.08m mol/g, even more preferably less than about 0.06m mol/g, in view of friction reduction benefit. It has been surprisingly found that higher levels of nitrogen (amine functional groups) in the amino silicone tend to result in less friction reduction, and consequently less conditioning benefit from the aminosilicone.

Aminosilicone useful herein are those which conform to the general formula (I):

(R₁)ₐG₃₋ₐ-Si-(-OSiG₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is an integer having a value from 1 to 3, preferably 1; b is 0, 1 or 2, preferably 1; n is a number from 1 to 2,000, preferably from 100 to 1,800, more preferably from 300 to 800, still more preferably 500-600; m is 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A⁻; -N(R₂)CH₂-CH₂-NR₂H₂A⁻; wherein R₂ is hydrogen, phenyl, benzyl, or a saturated hydrocarbon radical, preferably an alkyl radical from about C₁ to about C₂₀; A is a halide ion. L is preferably -N(CH₃)₂ or -NH₂, more preferably - NH₂.

One highly preferred amino silicones are those corresponding to formula (I) wherein m=0, a=1, q=3, G=methyl, n is preferably from about 1400 to about 1700, more preferably around 1600; and L is -N(CH₃)₂ or -NH₂, more preferably -NH₂. Another further highly preferred amino silicones are those corresponding to formula (I) wherein m=0, a=1, q=3, G=methyl, n is preferably from about 400 to about 800, more preferably about 500 to around 600; and L is -N(CH₃)₂ or -NH₂, more preferably -NH₂.

Such preferred aminosilicones can be called as terminal aminosilicones, as one or both ends of the silicone chain are terminated by nitrogen containing group. It is also surprisingly found that, such terminal aminosilicones provide improved friction reduction compared to graft aminosilicones.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from about 0.001% to about 10%, preferably up to about 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; coloring agents, such as any of the FD&C or D&C dyes; perfumes; and antidandruff agents such as zinc pyrithione and salicylic acid.

### PRODUCT FORMS

The hair care compositions of the present invention can be hair conditioning and/or hair styling products, and the like.

The hair care compositions of the present invention are, preferably, in the form of leave-on products, and can be formulated in a wide variety of product forms, including gels, sprays, oils, liquid and serum. Preferably, the compositions of the present invention are in a liquid form such as oil, liquid and serum.

### METHOD OF USE

For a leave-on form, the hair care composition is preferably applied to wet or damp hair prior to drying of the hair. After such hair care compositions are applied to the hair, the hair is dried and styled in accordance with the preference of the user. In the alternative, it may be applied to already dry hair, and the hair is then combed or styled, and dried in accordance with the preference of the user.

### EXAMPLES

The following examples further describe and demonstrate embodiments within the scope of the present invention. The examples are given solely for the purpose of illustration and are not to be construed as limitations of the present invention, as many variations thereof are possible Where applicable, ingredients are identified by chemical or CTFA name, or otherwise defined below.

### [Compositions]

| Components | Ex.1 | Ex.2 | Ex.3 | Ex.4 | Ex. i | Ex. ii | Ex.iii |
|---|---|---|---|---|---|---|---|
| Volatile isoparaffin *1 | 50.0 | 48.0 | 40.0 | 50.0 | 50.0 | 50.0 | 50.0 |
| First non-volatile mineral oil *2 | q.s. to 100% | q.s. to 100% | q.s. to 100% | q.s. to 100% | q.s. to 100% | - | - |
| Second non-volatile mineral oil *3 | 10.0 | 12.0 | 13.5 | 10.0 | - | q.s. to 100% | - |
| Non-volatile mineral oil *4 | - | - | - | - | - | - | q.s. to 100% |
| Aminosilicone *5 | 1.0 | 1.5 | 2.0 | - | 2.0 | 2.0 | 2.0 |
| Dimethicone Fluid *6 | - | - | - | 1.5 | - | - | - |
| Ester *7 | - | 2.0 | 20.0 | - | - | - | - |
| Perfume | 0.4 | 0.4 | 0.4 | 0.4 | 0.4 | 0.4 | 0.4 |
| Turbidity (NTU) at 25°C | 0.4 | < 2.0 | < 2.0 | < 2.0 | < 2.0 | > 5.0 | < 2.0 |
| Foaming | C1 | C1 | C | C1 | × | - | Δ |
| Stability | ○ | ○ | ○ | ○ | - | - | × |

| | | | | | | | |
|---|---|---|---|---|---|---|---|
| Definitions of Components *1 Volatile isoparaffin: Trimer of isobutene having a viscosity of 1.4 mm²· s⁻¹ at 37.8°C *2 First non-volatile mineral oil: Having a viscosity of about 11-14mm²·s⁻¹ at 37.8°C, and a relative density of about 0.820-0.831 at 25°C *3 Second non-volatile mineral oil: Having a viscosity of about 65-75mm²· s⁻¹ at 37.8°C, and a relative density of about 0.852-0.872 at 25°C *4 Non-volatile mineral oil: Having a viscosity of about 40mm²·s⁻¹ at 37.8°C, and a relative density of about 0.838-0.853 at 25°C *5 Aminosilicone: Terminal aminosilicone which is available from GE having a viscosity of about 10,000 mm²· s⁻¹ at 25°C, and having following formula: (R₁)ₐG₃₋ₐ-Si-(-OSiG₂)ₙ-(-OSiG_{b}(R₁)_{n-b})ₘ-(-O-SiG_{b-a}(R₁)ₐ wherein G is methyl; a is an integer of 1; n is a number from 400 to about 600; m is an integer of 0; R₁ is a monovalent radical conforming to the general formula CqH₂qL, wherein q is an integer of 3 and L is -NH₂ *6 Dimethicone Fluid: 15%/85% mixture of dimethicone having a viscosity of 18,000,000 mm²· s⁻¹ and dimethicone having a viscosity of 200 mm²· s⁻¹ at 25°C *7 Ester: Methy stearate | | | | | | | |

### Method of Preparation

The hair conditioning compositions of "Ex. 1" through "Ex. 4" and "Ex. i" through "Ex. iii" as shown above can be prepared by any conventional method well known in the art. They are suitably made as follows:
Silicone conditioning agents, and esters if included, are added to base oil with agitation at room temperature until homogenized. If included, other components such as perfumes are added to the mixture with agitation.

### Properties and Conditioning benefits

Turbidity is measured by the methods described above. For some of the above compositions, Foaming and/or Stability are evaluated by the following methods. The results of the evaluation are shown above.

### Foaming

Foaming is evaluated by 6 panelists, when applying 10ml of the composition.
C: Control
C1: Equal to Control
Δ: From 1 to 2 panelists answered that the composition caused foaming compared to Control.
× : From 3 to 6 panelists answered that the composition caused foaming compared to Control.

### Stability

Stability is evaluated by the composition appearance at 25°C, after its storage at 5 °C for 1 hour.
○: No phase separation was observed and/or Turbidity of below 2.0 was measured
× : Phase separation was observed and/or Turbidity of above 5.0 was measured

Examples 1 through 4 are hair conditioning compositions of the present invention which are particularly useful for leave-on use. The embodiments disclosed and represented by the previous "Ex. 1" through "Ex. 4" have many advantages. For example, they provide reduced foaming while providing improved dry conditioning by containing silicone conditioning agent. They also provide conditioned feel and reduced stickiness/greasiness, and have a clear product appearance. They also provide improved stability, especially improved silicone solubility at lower temperature.

Such advantages can be understood by the comparison between the examples of the present invention and comparative examples "Ex. i" through "Ex. iii". For example, the composition of the comparative example "Ex. i" has increased foaming, compared to the composition of "Ex. 3" of the present invention. For example, the composition of the comparative example "Ex. ii" does not have clear product appearance. For example, the compositions "Ex. 1" through "Ex. 4" of the present invention have improved stability compared to the composition of the comparative example "Ex. iii".

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made

## Claims

1. A non-aqueous hair care composition comprising by weight:
from 65% to 99.9% of a base oil consisting of:
a volatile isoparaffin;
a first non-volatile mineral oil having a viscosity of from 5mm²· s⁻¹ to 30mm² s⁻¹ at 37.8°C and a relative density of from 0.80 to 0.84 at 25°C; and
a second non-volatile mineral oil having a viscosity of from 45mm²· s⁻¹ to 100mm²· s⁻¹ at 37.8°C and a relative density of from 0.847 to 0.89 at 25°C;
from 0.1% to 15% of a non-volatile silicone conditioning agent having a viscosity of from 200 mm²·s⁻¹ at 25°C and
1% or less of water by weight of the composition.

2. The hair care composition of Claim 1, wherein the weight ratio of the volatile isoparaffin to a sum of the first and secondnon-volatile mineral oils is from 1:0.2 to 1:2.

3. The hair care composition of Claim 1, wherein the weight ratio of the first non-volatile mineral oil to the second non-volatile mineral oils is from 1:0.1 to 1:2.

4. The hair care composition of Claim 1, wherein the weight ratio of the first non-volatile mineral oil to the second non-volatile mineral oils is from 1:0.2 to 1:0.8.

5. The hair care composition of Claim 1, wherein the non-volatile silicone compound is an aminosilicone.

6. The hair care composition of Claim 5, wherein the aminosilicone having a formula:
(R1)ₐG₃₋ₐ-Si-(-OSiG₂)ₙ-(-OSiG_{b}(R₁)_{2-b})ₘ-O-SiG₃₋ₐ(R₁)ₐ
wherein G is hydrogen, phenyl, hydroxy, or C1-C8 alkyl; a is 1; n is a number from 100 to 1,800; m is 0; R1 is a monovalent radical conforming to the general formula CqH_{2q}L, wherein q is an integer having a value from 2 to 8 and L is -N(R₂)₂; wherein R₂ is hydrogen, phenyl, benzyl, or a saturated hydrocarbon radical; and A⁻ is a halide ion.

7. The hair care composition of Claim 6 wherein L is -N(CH₃)₂ or -NH₂.

8. The hair care composition of Claim 1 wherein the composition is free of surfactants.

9. The hair care composition of Claim 1, wherein the composition is for leave-on use.

10. The hair care composition of Claim 1, wherein the composition has a turbidity of 2.0NTU or less at 25°C.

## Patentansprüche

1. Nicht-wässrige Haarpflegezusammensetzung, die, bezogen auf das Gewicht, Folgendes umfasst:
zu 65 % bis 99,9 % eines Grundöls, bestehend aus:
einem flüchtigen Isoparaffin;
einem ersten nicht-flüchtigen Mineralöl mit einer Viskosität von etwa 5 mm²·s⁻¹ bis etwa 30 mm²·s⁻¹ bei 37,8 °C und einer relativen Dichte zwischen 0,80 und 0,84 bei 25°C; und
einem zweiten nicht-flüchtigen Mineralöl mit einer Viskosität von etwa 45 mm²·s⁻¹ bis etwa 100 mm²·s⁻¹ bei 37,8 °C und einer relativen Dichte zwischen 0,847 und 0,89 bei 25° C;
zwischen 0,1 % und 15 % eines nicht-flüchtigen Silikon-Konditioniermittel mit einer Viskosität zwischen 200 mm²·s⁻¹ bei 25° C und,
bezogen auf das Gewicht der Zusammensetzung, 1 % Wasser oder weniger.

2. Haarpflegezusammensetzung aus Anspruch 1, wobei das Gewichtsverhältnis des flüchtigen Isoparaffins zu einer Summe des ersten und des zweiten nicht-flüchtigen Mineralöls von 1:0,2 bis 1:2 beträgt.

3. Haarpflegezusammensetzung aus Anspruch 1, wobei das Gewichtsverhältnis des ersten nicht-flüchtigen Mineralöls zu dem zweiten nicht-flüchtigen Mineralöl von 1:0,1 bis 1:2 beträgt.

4. Haarpflegezusammensetzung nach Anspruch 1, wobei das Gewichtsverhältnis des ersten nicht-flüchtigen Mineralöls zu dem zweiten nicht-flüchtigen Mineralöl von 1:0,2 bis 1:0,8 beträgt.

5. Haarpflegezusammensetzung nach Anspruch 1, wobei es sich bei der Silikonverbindung um ein Aminosilicon handelt.

6. Haarpflegezusammensetzung nach Anspruch 5, wobei das Aminosilicon die folgende Formel aufweist:
(R₁)ₐG₃₋ₐ-Si-(-OSiG₂)ₙ-(-OSiG_{b}(R₁)_{2-b})ₘ-O-SiG₃₋ₐ(R₁)ₐ,
wobei es sich bei G um Wasserstoff-, Phenyl-, Hydroxy- oder C₁-C₈-Alkyl handelt; a gleich 1 ist; n eine Zahl zwischen 100 und 1.800 ist; m gleich 0 ist; R1 ein einwertiger Rest ist, der der allgemeinen Formel CqH₂qL entspricht, wobei q eine ganze Zahl ist, die einen Wert zwischen 2 und 8 hat und L gleich -N(R₂)₂ ist, wobei es sich bei R₂ um Wasserstoff-, Phenyl-, Benzyl- oder einen gesättigten Kohlenwasserstoffrest handelt; und A⁻ ein Halidion ist.

7. Haarpflegezusammensetzung nach Anspruch 6, wobei L -N(CH₃)₂ oder -NH₂ ist.

8. Haarpflegezusammensetzung nach Anspruch 1, wobei die Zusammensetzung keine Tenside enthält.

9. Haarpflegezusammensetzung nach Anspruch 1, wobei die Zusammensetzung nicht ausgespült werden muss.

10. Haarpflegezusammensetzung nach Anspruch 1, wobei die Zusammensetzung bei 25°C eine Trübung von 2,0 NTU oder weniger aufweist.

## Revendications

1. Composition non aqueuse pour soins capillaires comprenant, en poids :
de 65 % à 99,9 % d'une base huileuse constituée :
d'une isoparaffine volatile ;
d'une première huile non-volatile ayant une viscosité allant d'environ 5 mm²·s⁻¹ à environ 30 mm²·s⁻¹ à 37,8 °C et une densité relative allant de 0,80 à 0,84 à 25°C; et
d'une première huile minérale non-volatile ayant une viscosité allant de 45 mm²·s⁻¹ à 100 mm²·s⁻¹ à 37,8 °C et une densité relative allant de 0,847 à 0,89 à 25°C;
de 0,1 % à 15 % d'un agent de conditionnement siliconé non-volatil ayant une viscosité allant de 200 mm²·s⁻¹ à 25°C et
1 % ou moins d'eau en poids de la composition.

2. Composition pour soins capillaires selon la revendication 1, dans laquelle le rapport massique d'isoparaffine volatile par rapport à la somme des premières et secondes huiles minérales non-volatiles va de 1:0,2 à 1:2.

3. Composition pour soins capillaires selon la revendication 1, dans laquelle le rapport massique entre la première huile minérale non-volatile et les secondes huiles minérales non-volatiles va de 1:0,1 à 1:2.

4. Composition pour soins capillaires selon la revendication 1, dans laquelle le rapport massique entre la première huile minérale non-volatile et les secondes huiles minérales non-volatiles va de 1:0,2 à 1:0,8.

5. Composition pour soins capillaires selon la revendication 1, dans laquelle le composé siliconé non-volatil est une aminosilicone.

6. Composition pour soins capillaires selon la revendication 5, dans laquelle l'aminosilicone est de la formule suivante :
(R₁)ₐG₃₋ₐ-Si-(-OSiG₂)ₙ-(-OSiG_{b}(R₁)_{2-b})ₘ-O-SiG₃₋ₐ(R₁)ₐ
dans laquelle G est hydrogène, phényle, hydroxy ou alkyle en C₁-C₈ ; a est 1 ; n est un nombre allant de 100 à 1 800 ; m est 0 ; R1 est un radical monovalent suivant la formule générale CqH₂qL, dans laquelle q est un entier ayant une valeur allant de 2 à 8 et L est -N(R₂)₂ ; dans laquelle R₂ est hydrogène, phényle, benzyle ou un radical hydrocarbure saturé ; et A⁻ désigne un ion halogénure.

7. Composition pour soins capillaires selon la revendication 6, dans laquelle Lest -N(CH₃)₂ ou -NH₂.

8. Composition pour soins capillaires selon la revendication 1, dans laquelle la composition est dépourvue d'agents tensioactifs.

9. Composition pour soins capillaires selon la revendication 1, dans laquelle la composition est destinée à ne pas être rincée.

10. Composition pour soins capillaires selon la revendication 1, dans laquelle la composition a une turbidité de 2,0 NTU ou moins à 25°C.
